# EUROPEAN PATENT SPECIFICATION

(11) **EP 1 747 006 B1**
(45) Date of publication and mention of the grant of the patent: **01.07.2009**
(21) Application number: 05709167.0
(22) Date of filing: 19.01.2005
(51) Int. Cl.: A61K 31/726, A61K 31/727, A61K 31/728, A61K 31/196, A61K 31/18, A61K 31/405, A61P 1/04

(54) **COMPOSTIONS COMPRISING GLYCOSAMINOGLYCAN AND NONSTEROIDAL ANTI-INFLAMMATORY DRUG**
ZUSAMMENSETZUNGEN AUS EINEM GLYCOSAMINOGLYKAN UND EINEM NICHTSTEROIDALEN ANTIRHEUMATIKUM
COMPOSITIONS COMPRENANT DU GLYCOSAMINOGLYCANE ET MEDICAMENT ANTI-INFLAMMATOIRE NONSTEROIDIEN

(30) Priority: 20.01.2004 IN DE01022004
(43) Date of publication of application: 31.01.2007
(73) Proprietor: Panacea Biotec Ltd., New Delhi 110 044 (IN)
(72) Inventor: JAIN, Rajesh, c/o Panacea Biotec Ltd, New Delhi 110 044 (IN); JINDAL, Kour, Chand, c/o Panacea Biotec Ltd, New Delhi 110 044 (IN); SINGH, Sukhjeet, c/o Panacea Biotec Ltd, New Delhi 110 044 (IN)
(74) Representative: Matthews, Derek Peter
(86) International application number: PCT/IN2005/000026
(87) International publication number: WO 2005/067943

(56) References cited:
- EP-B- 0 433 817
- US-A- 5 668 119
- US-A- 5 972 906
- PATENT ABSTRACTS OF JAPAN vol. 1997, no. 07, 31 July 1997 (1997-07-31) & JP 09 087174 A (KOBAYASHI PHARMACEUT CO LTD), 31 March 1997 (1997-03-31)
- HORI Y ET AL: "EFFECTS OF CHONDROITIN SULFATE ON COLITIS INDUCED BY DEXTRAN SULFATE SODIUM IN RATS" JAPANESE JOURNAL OF PHARMACOLOGY, THE JAPANESE PHARMACOLOGICAL SOCIETY, KYOTO, JP, vol. 85, no. 2, February 2001 (2001-02), pages 155-160, XP008015568 ISSN: 0021-5198 cited in the application
- LYONS K C ET AL: "Changes in sulfated macromolecules produced in vivo during normal and indomethacin-delayed ulcer healing in rats." DIGESTIVE DISEASES AND SCIENCES. AUG 1997, vol. 42, no. 8, August 1997 (1997-08), pages 1755-1764, XP000326236 ISSN: 0163-2116
- WRENSHALL L E ET AL: "Regulation of murine splenocyte responses by heparan sulfate" JOURNAL OF IMMUNOLOGY 1991 UNITED STATES, vol. 147, no. 2, 1991, pages 455-459, XP002326237 ISSN: 0022-1767

## Description

### Field of the invention

The present invention relates to novel pharmaceutical compositions comprising specific glycosaminoglycans or salts thereof, preferably chondroitin or salts thereof, more preferably chondroitin sulphate, and specific nonsteroidal anti-inflammatory drug(s) or salts thereof, optionally with pharmaceutically acceptable excipient(s). The compositions of the present invention provide gastrosparing effect in conditions where nonsteroidal anti-inflammatory drug(s) or their salts are used, particularly in mammals. The present invention also relates to a process for the manufacture of such novel compositions and a method to minimize the nonsteroidal anti-inflammatory drug(s) induced gastric toxicity.

### Background of the invention

Glycosaminoglycans are a type of long, unbranched polysaccharide molecule. They are major structural components of cartilage and are also found in the cornea of the eye. Examples of common glycosaminoglycans include keratan sulphate, chondroitin sulphate, dermatan sulphate, heparin sulphate, and hyaluronan.

Chondroitin sulphate is a sulphated glycosaminoglycan that consists of repeating chains of glycosaminoglycan molecules. It is a sulphated linear polysaccharide constructed of two or three kinds of monosaccharides namely N-acetylgalactosamine, L-iduronic acid, and D-glucuronic acid. It is present in various connective tissues, including cartilage, skin, blood vessels, and bone (Hardingham and Fosang, 1992). It is the major constituent of articular cartilage proteoglycan and plays an important role in the elasticity and function of articular cartilage (Hardingham and Bayliss, 1992). It has been used clinically as a chondroprotective agent for treatment of osteoarthritis (Morreale et al., 1996; Bucsi and Poor, 1998). Chondroitin sulphate is currently marketed and therapeutically prescribed for neurodynia, lumbago and arthrodynia as a cartilage protective agent.

Hori et al. demonstrated the therapeutic efficacy of chondroitin sulphate in protection of digestive mucosa against animal model of inflammatory bowel disease (Hori et al., 2001).

Nonsteroidal anti-inflammatory drugs (NSAIDs) are widely used in the management of pain and inflammation. They are among the most commonly prescribed drugs in the world but their therapeutic effects are limited by their untoward effects on the gastrointestinal tract (Kulkarni and Varghese, 1998; Kulkarni et al., 2000). NSAIDs act by non-selectively inhibiting cyclooxygenase (COX) enzyme thereby blocking prostaglandins release. Two isoforms of COX have been identified namely COX-1 and COX-2. COX-1 is constitutive and involves in house keeping functions such as mucus secretion and renal blood flow; whereas COX-2 is inducible by pain and inflammatory stimulus (Kulkarni et al., 2000). The classical NSAIDs inhibit both COX isoforms thereby blocking the useful gastric prostaglandins released by gastric COX-1 leading to gastrointestinal side effects ranging from dyspepsia to life threatening gastrointestinal bleeding and potentially perforating gastro-duodenal ulcers (Garcia Rodriguez and Jick, 1994; Langman et al., 1994).

Various attempts have been made to develop NSAIDs with reduced gastrointestinal side effects which has led to the development of several types of NSAIDs such as selective inhibitors of COX-2 (Brideau et al., 1999), nitric oxide releasing NSAIDs (Takeuchi et al., 1998), NSAIDs pre-associated with zwitter ionic phospholipids (Wallace and Chin, 1997), and NSAIDs complexation with divalent metal ions (Dendrinu-Samara et al., 1998). Of these attempts, the main approach has been to develop safer NSAIDs on the basis of the COX-1 hypothesis.

There still exists an unmet need to develop new combination products or new methods to minimize the NSAID(s) induced gastric toxicity. No composition has been reported in the art where chondroitin or salts thereof, especially chondroitin sulphate is employed in combination with NSAID(s) to minimize the NSAID(s) induced gastric toxicity. The present invention provides novel pharmaceutical compositions comprising specific glycosaminoglycans or salts thereof, such as chondroitin sulphate and specific nonsteroidal anti-inflammatory drug(s).

### Objective of the invention

It is an objective of the present invention to provide a novel pharmaceutical composition comprising specific glycosaminoglycans or salts thereof and at least one nonsteroidal anti-inflammatory drug or salts thereof optionally with pharmaceutically acceptable excipients, wherein the said composition provides a gastrosparing effect and minimizes the gastric toxicity induced by the administration of nonsteroidal anti-inflammatory drug.

It is an objective of the present invention to provide a pharmaceutical composition comprising a glycosaminoglycan selected from a group consisting of chondroitin sulphate, keratan sulphate, dermatan sulphate and salts thereof, and at least one nonsteroidal anti-inflammatory drug selected from a group consisting of indomethacin, flurbiprofen, naproxen, diclofenac, ketorolac, mefenamic acid, ketoprofen, meloxicam, piroxicam, nimesulide, celecoxib, rofecoxib, etoricoxib, parecoxib, valdecoxib, lumiracoxib, licofetone and salts thereof, optionally with pharmaceutically acceptable excipients, wherein the said compositions provides a gastrosparing effect and minimizes the gastric toxicity induced by the administration of the said nonsteroidal anti-inflammatory drug.

It is another objective of the invention to provide a process for preparing such pharmaceutical composition which comprises mixing of glycosaminoglycan or salts thereof and at least one nonsteroidal anti-inflammatory drug or salts thereof optionally with pharmaceutically acceptable excipients and formulating into a suitable dosage form.

The present invention also relates to a method of providing a gastrosparing effect and minimizing the gastric toxicity induced by the administration of nonsteroidal anti-inflammatory drug by administering a pharmaceutical composition comprising specific glycosaminoglycan or salts thereof and at least one nonsteroidal anti-inflammatory drug or salts thereof optionally with pharmaceutically acceptable excipients.

### Detailed description of the invention

The present invention provides a pharmaceutical composition comprising a glycosaminoglycan selected from a group consisting of chondroitin sulphate, keratan sulphate, dermatan sulphate and salts thereof, and at least one nonsteroidal anti-inflammatory drug selected from a group consisting of indomethacin, flurbiprofen, naproxen, diclofenac, ketorolac, mefenamic acid, ketoprofen, meloxicam, piroxicam, nimesulide, celecoxib, rofecoxib, etoricoxib, parecoxib, valdecoxib, lumiracoxib, licofetone and salts thereof, optionally with pharmaceutically acceptable excipients, wherein the said compositions provides a gastrosparing effect and minimizes the gastric toxicity induced by the administration of the said nonsteroidal anti-inflammatory drug.

In an embodiment, the invention provides novel pharmaceutical compositions comprising of chondroitin or salts thereof, preferably chondroitin sulphate, and nonsteroidal anti-inflammatory drug(s) or salts thereof, optionally with pharmaceutically acceptable excipient(s).

In an embodiment of the present invention, the ratio of glycosaminoglycan or salts thereof and nonsteroidal anti-inflammatory drug or salts thereof in the composition is from 100:1 to 1:100.

In an embodiment of the present invention, the pharmaceutically acceptable excipients are selected from a group comprising diluents, disintegrants, fillers, bulking agents, vehicles, pH adjusting agents, stabilizers, anti-oxidants, binders, buffers, lubricants, antiadherants, coating agents, preservatives, emulsifiers, suspending agents, release controlling agents, polymers, colorants, flavoring agents, plasticizers, solvents, preservatives, glidants and chelating agents; used either alone or in combination thereof.

The pharmaceutical compositions of the present invention may be administered in the form of conventional pharmaceutical compositions, and may be formulated by means known in the art. The compositions of the present invention can be formulated as oral dosage forms such as tablets, pills, capsules, gels, finely divided powders, dispersions, suspensions, solutions, emulsions; pulmonary and nasal dosage form such as sprays, aerosols; topical dosage forms such as gels, ointments, creams; parenteral dosage forms; controlled release formulations; fast melt formulations, lyophilized formulations, delayed release formulations, sustained release, extended release formulations, pulsatile release formulations, and mixed immediate release and controlled release formulations. In an embodiment, the composition of the present invention is provided to be taken orally by way of a pediatric suspension, capsule, or tablet.

The present invention also relates to a method to minimize the nonsteroidal anti-inflammatory drug(s) induced gastric toxicity. In an embodiment of the present invention, the compositions comprising specific glycosaminoglycans or salts thereof and nonsteroidal anti-inflammatory drug(s) or salts thereof is intended to reduce the incidence of dyspepsia, heartburn, bleeding ulcers, and other gastrointestinal complications associated with the use of nonsteroidal anti-inflammatory drug(s).

In yet another embodiment, the present invention also provides process for the manufacture of analgesic and/or anti-inflammatory compositions comprising glycosaminoglycan, preferably chondroitin sulphate or salts thereof and nonsteroidal anti-inflammatory drug(s) or salts thereof. The process for preparing such pharmaceutical composition comprises mixing of glycosaminoglycan or salts thereof and at least one nonsteroidal anti-inflammatory drug or salts thereof optionally with pharmaceutically acceptable excipients and formulating into a suitable dosage form.

The novel pharmaceutical compositions of the present invention are also intended to ensure greater patient compliance and a promising potential to reduce the gastric toxicity associated with use of nonsteroidal anti-inflammatory drug(s). Furthermore, the novel pharmaceutical compositions of present invention are not expected to alter the pharmacodynamic profile of either glycosaminoglycan or nonsteroidal anti-inflammatory drug(s).

The nonsteroidal anti-inflammatory drug(s) of the present invention includes indomethacin, flurbiprofen, naproxen, diclofenac, ketorolac, mefenamic acid, ketoprofen, meloxicam, piroxicam, nimesulide, celecoxib, rofecoxib, etoricoxib, parecoxib, valdecoxib, lumiracoxib, licofelone, or salts thereof.

Examples of common glycosaminoglycans that may be used include keratan sulphate, chondroitin sulphate, dermatan sulphate, heparin sulphate, and hyaluronan.

By careful experimentation, the inventors have found that among the nonsteroidal anti-inflammatory drugs (NSAIDs), Indomethacin and Diclofenac produced severe gastric damage in the stomach. In the present invention, it was surprisingly found that combining Chondroitin sulphate with Indomethacin or Diclofenac significantly attenuated Indomethacin or Diclofenac *per se* induced gastric ulcerations and perforations in rats; thus producing unexpected gastric protection by minimizing the NSAID induced gastric toxicity.

### Determination of Biological activity

### NSAID-induced acute gastric ulcers in rats

The gastroprotective effect of Chondroitin sulphate was studied on NSAID-induced acute gastric ulcers in rat. The NSAIDs used for the study are Indomethacin and Diclofenac free acid.

### Indomethacin-induced acute gastric ulcers in rats

The observed unexpected gastroprotective effect of Chondroitin sulphate is evidenced by test conducted in rats. Wistar rats of either sex were procured from Central Animal House facility, Panacea Biotec Ltd., India. The animals were fasted overnight and those weighing between 150-170 g at the time of testing were used throughout. All animals were dosed sequentially by the oral route with 0.5% carboxy methylcellulose (CMC) suspension of Indomethacin and/or solutions of Chondroitin sulphate in distilled water. A dosing volume of 10 ml/kg was used for each sequential solution or suspension.

The ulcers were induced as described by Chan et. al. (1995). In brief, overnight fasted animals were administered orally with 20 mg/kg of Indomethacin on the day of experimentation. Chondroitin sulphate was administered at a dose of 50 or 100 mg/kg, 15 minutes before Indomethacin administration. The animals were sacrificed after 4 hours of Indomethacin administration; the stomach was opened along the greater curvature and washed under running water. The number of ulcers was identified using 10X magnifying lens, counted, multiplied by respective score, and summed up for each animal. The mean score as Ulcer index in each group was calculated and compared with Indomethacin group.

| **Ulcer scoring:** | |
|---|---|
| **Size (longest diameter)** | **Score** |
| Ulcers < 1 mm | 1 |
| Ulcers 1 - 3 mm | 5 |
| Ulcers > 3 mm | 10 |

The results of the study are presented in Table 1.

**Table 1: Effect of Chondroitin sulphate on Indomethacin-induced gastric ulcers in rats**

| S. No. | Treatment (mg/kg, p.o.) | Ulcer Index |
|---|---|---|
| 1 | Vehicle control (0.5% CMC) | 2.25 ± 0.48 |
| 2 | Indomethacin control (20) | 66.25 ± 15.8^{a} |
| 3 | Chondroitin sulphate (50) + Indomethacin (20) | 23.0 ± 8.04* |
| 4 | Chondroitin sulphate (100) + Indomethacin (20) | 6.0 ± 3.07* |

| | | |
|---|---|---|
| All the values are expressed as mean ± S.E.M. (Standard Error of Mean). n (no. of rats) = 6 - 9 in each treatment group; *p < 0.05 as compared to control (vehicle) (*t*-test); ^{a}p < 0.05 as compared to *per se* treatments (ANOVA followed by Dunnett's test). Single oral administration of indomethacin 20 mg/kg produced severe gastric damage (red colored, bleeding ulcers and disruption of mucus layer) in fasted animals with ulcer score of 66.25 ± 15.8 when compared to the control value of 2.25 ± 0.48. Oral coadministration of Chondroitin Sulphate 50 mg/kg or 100 mg/kg with Indomethacin 20 mg/kg showed unexpected gastroprotection with ulcer scores of 23.0 ± 8.04 and 6.0 ± 3.07, respectively. Also the extent of gastroprotection was dose dependent as evident from the ulcer index values for 50 and 100 mg/kg of Chondroitin sulphate administered together with 20 mg/kg of Indomethacin (Table 1). | | |

### Diclofenac-induced acute gastric ulcers in rats

Wistar rats of either sex were fasted overnight and weighing 170 - 200 g at the time of testing were used throughout. All animals were dosed sequentially by the oral route with 0.5% carboxy methylcellulose suspension of diclofenac free acid and/or solutions of chondroitin sulphate in distilled water. A dosing volume of 10 ml/kg was used for each sequential solution or suspension. All doses were coded and the test was performed under a code not known to the observer.

The ulcers were induced as described by Chan *et al.* (1995). In brief, overnight fasted animals were weighed and randomly divided into four groups. Three groups of animals were administered orally with 100 mg/kg of diclofenac free acid and the last group received equivalent volume of vehicle alone on the day of experimentation. Two groups of animals received chondroitin sulphate at a dose of 50 or 100 mg/kg 15 min before diclofenac free acid administration. The animals were sacrificed 4 h after diclofenac free acid administration; the stomach was opened along the greater curvature, and washed under running water. The number of ulcers was identified using 10X magnifying lens, counted, multiplied by respective score, and summed up for each animal. The mean score as ulcer index in each group was calculated as mentioned below.

| **Ulcer scoring:** | |
|---|---|
| **Size (longest diameter)** | **Score** |
| Ulcers < 1 mm | 1 |
| Ulcers 1 - 3 mm | 5 |
| Ulcers > 3 mm | 10 |

The results of the study are presented in Table 2.

**Table 2: Effect of chondroitin sulphate on diclofenac-induced gastric ulcers in rats**

| S. No. | Treatment (mg/kg, p.o.) | Ulcer Index |
|---|---|---|
| 1 | Vehicle control (0.5% CMC) ; | 1.0 ± 0.63 |
| 2 | Diclofenac control (100) | 57.50 ± 11.30^{a} |
| 3 | Chondroitin sulphate (50) + Diclofenac (100) | 19.80 ± 5.52* |
| 4 | Chondroitin sulphate (100) + Diclofenac (100) | 12.00 ± 4.71 * |

| | | |
|---|---|---|
| All the values are expressed as mean ± SEM. n = 5 to 7 in each treatment; ^{a}p < 0.05 as compared to vehicle control; *p < 0.05 as compared to diclofenac control. | | |

Single oral administration of diclofenac free acid 100 mg/kg produced severe gastric damage (red colored, bleeding ulcers and disruption of mucus layer) in fasted animals when compared to the control animals (Table 1). Oral administration of chondroitin sulphate 50 mg/kg or 100 mg/kg 15 min before the administration of diclofenac free acid 100 mg/kg showed gastroprotection as evidenced by significant reduction in ulcer index as compared to diclofenac free acid control (Table 2).

The examples given below serve to illustrate embodiments of the present invention. However they do not intend to limit the scope of present invention.

### EXAMPLES

### Example 1 (Capsule)

| **Ingredient** | **mg/capsule** |
|---|---|
| Chondroitin sulphate | 200.0 |
| Indomethacin | 100.0 |
| Microcrystalline cellulose | 200.8 |
| Mannitol | 72.0 |
| Talc | 3.2 |
| Sodium starch glycollate | 12.0 |
| Colloidal silicon dioxide | 12.0 |

### Procedure:

1) Chondroitin sulphate, Indomethacin, Microcrystalline cellulose and Mannitol are sifted and mixed together.
2) Talc, Sodium starch glycollate and Colloidal silicon dioxide are passed through fine sieves individually and then mixed together.
3) the materials of step 1 and 2 are mixed and filled into empty hard gelatin capsules

### Example 2 (Uncoated tablet)

| **Ingredient** | **mg/tablet** |
|---|---|
| Chondroitin sulphate | 200.0 |
| Diclofenac free acid | 50.0 |
| Microcrystalline cellulose | 120.0 |
| Mannitol | 80.0 |
| Croscarmellose sodium | 10.0 |
| Lactose | 66.0 |
| Talc | 4.0 |
| Colloidal silicon dioxide | 10.0 |
| Croscarmellose sodium | 10.0 |

### Procedure:

1) Chondroitin sulphate, Diclofenac free acid, Microcrystalline cellulose, Mannitol, Croscarmellose sodium and Lactose are sifted and mixed together.
2) The material of step 1 is compacted.
3) The compacts of step 2 are passed through sieve and mixed.
4) Talc, Colloidal silicon dioxide and Croscarmellose sodium are passed through fine sieve and mixed together.
5) The material of step 3 is mixed with material of step 4.
6) The material of step 5 is compressed into tablets.

### Example 3 (Uncoated tablet)

| **Ingredient** | **mg/tablet** |
|---|---|
| Chondroitin sulphate | 400.0 |
| Nimesulide | 100.0 |
| Microcrystalline cellulose | 120.0 |
| Mannitol | 80.0 |
| Croscarmellose sodium | 10.0 |
| Lactose | 66.0 |
| Talc | 4.0 |
| Colloidal silicon dioxide | 10.0 |
| Croscarmellose sodium | 10.0 |

### Procedure:

1) Chondroitin sulphate, Nimesulide, Microcrystalline cellulose, Mannitol, Croscarmellose sodium and Lactose are sifted and mixed together.
2) The material of step 1 is compacted.
3) The compacts of step 2 are passed through sieve and mixed.
4) Talc, Colloidal silicon dioxide and Croscarmellose sodium are passed through fine sieve and mixed together.
5) The material of step 3 is mixed with material of step 4.
6) The material of step 5 is compressed into tablets.

### Example 4 (Uncoated tablet)

| **Ingredient** | **mg/tablet** |
|---|---|
| Dermatan sulphate | 100.0 |
| Diclofenac sodium | 75.0 |
| Microcrystalline cellulose | 120.0 |
| Mannitol | 80.0 |
| Croscarmellose sodium | 10.0 |
| Lactose | 66.0 |
| Talc | 4.0 |
| Colloidal silicon dioxide | 10.0 |
| Croscarmellose sodium | 10.0 |

### Procedure:

1) Dermatan sulphate, Diclofenac sodium, Microcrystalline cellulose, Mannitol, Croscarmellose sodium and Lactose are sifted and mixed together.
2) The material of step 1 is compacted.
3) The compacts of step 2 are passed through sieve and mixed.
4) Talc, Colloidal silicon dioxide and Croscarmellose sodium are passed through fine sieve and mixed together.
5) The material of step 3 is mixed with material of step 4.
6) The material of step 5 is compressed into tablets.

### Example 5 (Uncoated tablet)

| **Ingredient** | **mg/tablet** |
|---|---|
| Chondroitin sulphate | 200.0 |
| Nimesulide | 100.0 |
| Microcrystalline cellulose | 120.0 |
| Mannitol | 80.0 |
| Croscarmellose sodium | 10.0 |
| Lactose | 66.0 |
| Talc | 4.0 |
| Colloidal silicon dioxide | 10.0 |
| Croscarmellose sodium | 10.0 |

### Procedure:

1) Chondroitin sulphate, Nimesulide, Microcrystalline cellulose, Mannitol, Croscarmellose sodium and Lactose are sifted and mixed together.
2) The material of step 1 is compacted.
3) The compacts of step 2 are passed through sieve and mixed.
4) Talc, Colloidal silicon dioxide and Croscarmellose sodium are passed through fine sieve and mixed together.
5) The material of step 3 is mixed with material of step 4.
6) The material of step 5 is compressed into tablets.

### Example 6 (Film-coated tablet)

| **Ingredient** | **mg/tablet** |
|---|---|
| Core tablet composition | |
| Chondroitin sulphate | 200.0 |
| Diclofenac sodium | 75.0 |
| Microcrystalline cellulose | 120.0 |
| Mannitol | 80.0 |
| Croscarmellose sodium | 10.0 |
| Lactose | 66.0 |
| Talc | 4.0 |
| Colloidal silicon dioxide | 10.0 |
| Croscarmellose sodium | 10.0 |
| | |

| Film coating composition | |
|---|---|
| Hydroxypropylmethyl cellulose (E-15) | 12.0 |
| Polyethylene glycol 400 (PEG 400) | 2.4 |
| Iron oxide red | 0.75 |
| Iron oxide yellow | 0.50 |
| Titanium dioxide | 0.25 |
| Isopropyl alcohol | q.s. (lost in processing) |
| Dichloromethane | q.s. (lost in processing) |

### Procedure:

1) Chondroitin sulphate, Diclofenac sodium, Microcrystalline cellulose, Mannitol, Croscarmellose sodium and Lactose are sifted and mixed together.
2) The material of step 1 is compacted.
3) The compacts of step 2 are passed through sieve and mixed.
4) Talc, Colloidal silicon dioxide and Croscarmellose sodium are passed through fine sieve and mixed together.
5) The material of step 3 is mixed with material of step 4.
6) The material of step 5 is compressed into tablets.
7) Hydroxypropylmethyl cellulose is dispersed in a mixture of Isopropyl alcohol and Dichloromethane with continuous mixing in homogenizer.
8) Polyethylene glycol 400 is added to the above solution of step 7 and mixed.
9) Iron oxide red, Iron oxide yellow and Titanium dioxide are passed through fine sieve and mixed.
10) The material of step 9 is added to material of step 8 and mixed for 30 minutes.
11) The core tablets are charged into the coating pan and coated with the coating solution of step 10 till an average tablet weight gain of ~2-3% is achieved.

## Claims

1. A pharmaceutical composition comprising a glycosaminoglycan selected from a group consisting of chondroitin sulphate, keratan sulphate, dermatan sulphate and salts thereof, and at least one nonsteroidal anti-inflammatory drug selected from a group consisting of indomethacin, flurbiprofen, naproxen, diclofenac, ketorolac, mefenamic acid, ketoprofen, meloxicam, piroxicam, nimesulide, celecoxib, rofecoxib, etoricoxib, parecoxib, valdecoxib, lumiracoxib, licofetone and salts thereof, optionally with pharmaceutically acceptable excipients, wherein the said compositions provides a gastrosparing effect and minimizes the gastric toxicity induced by the administration of the said nonsteroidal anti-inflammatory drug.

2. A composition according to claim 1, wherein the nonsteroidal anti-inflammatory drug is selected from a group consisting of indomethacin, diclofenac, nimesulide and salts thereof.

3. A composition according to claim 1, wherein the ratio of glycosaminoglycan or salts thereof and nonsteroidal anti-inflammatory drug or salts thereof is from 100:1 to 1:100.

4. A composition according to any of claims 1 to 3, wherein the pharmaceutically acceptable excipients are selected from a group comprising diluents, disintegrants, fillers, bulling agents, vehicles, pH adjusting agents, stabilizers, anti-oxidants, binders, buffers, lubricants, antiadherants, coating agents, emulsifiers, suspending agents, release controlling agents, polymers, colorants, flavoring agents, plasticizers, solvents, preservatives, glidants, chelating agents, used either alone or in combination thereof.

5. A composition according to any of claims 1 to 4, which is formulated as an oral dosage form, a pulmonary dosage form, a nasal dosage form, a topical dosage form, a parenteral dosage form, a controller release formulation, a fast melt formulation, a lyophilized formulation, a delayed release formulation, a sustained release formulation, an extended release formulation, a pulsatile release formulation or a mixed immediate release and controlled release formulation.

6. A composition according to claim 5, which is formulated as a tablet, pill, capsule, gel, finely divided powder, dispersion, suspension, solution or emulsion.

7. A process for preparing a pharmaceutical composition according to claim 1, which comprises mixing a glycosaminoglycan selected from a group consisting of chondroitin sulphate, keratan sulphate, dermatan sulphate and salts thereof and at least one nonsteroidal anti-inflammatory drug selected from a group consisting of indomethacin, flurbiprofen, naproxen, diclofenac, ketorolac, mefenamic acid, ketoprofen, meloxicam, piroxicam, nimesulide, celecoxib, rofecoxib, etoricoxib, parecoxib, valdecoxib, lumiracoxib, licofetone and salts thereof, with pharmaceutically acceptable excipients and formulating into a suitable dosage form.

8. A process according to claim 7, wherein the ratio of glycosaminoglycan or salts thereof and nonsteroidal anti-inflammatory drug or salts thereof is from 100:1 1 to 1:100.

9. Use of a combination comprising a glycosaminoglycan selected from a group consisting of chondroitin sulphate, keratan sulphate, dermatan sulphate and salts thereof and at least one nonsteroidal anti-inflammatory drug selected from a group consisting of indomethacin, flurbiprofen, naproxen, diclofenac, ketorolac, mefenamic acid, ketoprofen, meloxicam, piroxicam, nimesulide, celecoxib, rofecoxib, etoricoxib, parecoxib, valdecoxib, lumiracoxib, licofetone and salts thereof, optionally with pharmaceutically acceptable excipients, in the preparation of a composition providing a gastrosparing effect and minimizing the gastric toxicity induced by the administration of nonsteroidal anti-inflammatory drug.

## Patentansprüche

1. Pharmazeutische Zusammensetzung enthaltend ein Glykosaminoglykan, das ausgewählt ist aus einer Gruppe bestehend aus Chondroitinsulfat, Keratansulfat, Dermatansulfat und Salzen davon, und mindestens einen nicht steroidalen entzündungshemmenden Wirkstoff, der ausgewählt ist aus einer Gruppe bestehend aus Indomethacin, Flurbiprofen, Naproxen, Diclofenac, Ketorolac, Mefenaminsäure, Ketoprofen, Meloxicam, Piroxicam, Nimesulid, Celecoxib, Rofecoxib, Etoricoxib, Parecoxib, Valdecoxib, Lumiracoxib, Licofelon und Salzen davon, optional mit unter pharmazeutischen Gesichtspunkten akzeptablen Hilfsstoffen, wobei die Zusammensetzung einen magenschonenden Effekt zur Verfügung stellt und die durch die Verabreichung des nicht steroidalen entzündungshemmenden Wirkstoffs hervorgerufene Gastrotoxizität minimiert.

2. Zusammensetzung gemäß Anspruch 1, worin der nicht steroidale entzündungshemmende Wirkstoff ausgewählt ist aus einer Gruppe bestehend aus Indomethacin, Diclofenac, Nimesulid und Salzen davon.

3. Zusammensetzung gemäß Anspruch 1, worin das Verhältnis von Glykosaminoglykan oder Salzen davon und nicht steroidalem entzündungshemmenden Wirkstoff oder Salzen davon von 100:1 bis 1:100 beträgt.

4. Zusammensetzung gemäß einem der Ansprüche 1 bis 3, worin die unter pharmazeutischen Gesichtspunkten akzeptablen Hilfsstoffe ausgewählt sind aus einer Gruppe umfassend Verdünner, Sprengmittel, Füller, Füllstoffe, Vehikel, den pH-Wert regulierende Agenzien, Stabilisatoren, Antioxidantien, Binder, Puffer, Gleitmittel, Antihaftmittel, Überzugsmittel, Emulgatoren, Suspendiermittel, die Freisetzung steuernde Agenzien, Polymere, Färbemittel, Aromatisierungsmittel, Weichmacher, Lösungsmittel, Konservierungsmittel, Fließverbesserer, Chelatbildner, die entweder einzeln oder in Kombination miteinander eingesetzt werden.

5. Zusammensetzung gemäß einem der Ansprüche 1 bis 4, die formuliert ist als eine orale Verabreichungsform, eine pulmonale Verabreichungsform, eine nasale Verabreichungsform, eine topische Verabreichungsform, eine parenterale Verabreichungsform, eine Formulierung mit kontrollierter Freisetzung, eine schnell schmelzende Formulierung, eine lyophilisierte Formulierung, eine Formulierung mit verzögerter Freisetzung, eine Formulierung mit anhaltender Freisetzung bzw. eine Sustained-Release Formulierung, eine Formulierung mit langanhaltender Freisetzung bzw. eine Extended-Release Formulierung, eine Formulierung mit stoßweiser bzw. pulsierender Freisetzung oder eine Formulierung, die unmittelbare Freisetzung und kontrollierte Freisetzung verbindet.

6. Zusammensetzung gemäß Anspruch 5, die formuliert ist als eine Tablette, eine Pille, eine Kapsel, ein Gel, ein fein verteiltes Pulver, eine Dispersion, eine Suspension, eine Lösung oder eine Emulsion.

7. Verfahren zur Herstellung einer pharmazeutischen Zusammensetzung gemäß Anspruch 1, umfassend das Vermischen eines Glykosaminoglykans, das ausgewählt ist aus einer Gruppe bestehend aus Chondroitinsulfat, Keratansulfat, Dermatansulfat und Salzen davon, und mindestens eines nicht steroidalen entzündungshemmenden Wirkstoffs, der ausgewählt ist aus einer Gruppe bestehend aus Indomethacin, Flurbiprofen, Naproxen, Diclofenac, Ketorolac, Mefenaminsäure, Ketoprofen, Meloxicam, Piroxicam, Nimesulid, Celecoxib, Rofecoxib, Etoricoxib, Parecoxib, Valdecoxib, Lumiracoxib, Licofelon und Salzen davon, mit unter pharmazeutischen Gesichtspunkten akzeptablen Hilfsstoffen und das Formulieren in eine geeignete Verabreichungsform.

8. Verfahren gemäß Anspruch 7, bei dem das Verhältnis von Glykosaminoglykan oder Salzen davon und nicht steroidalem entzündungshemmenden Wirkstoff oder Salzen davon von 100:1 bis 1:100 beträgt.

9. Verwendung einer Kombination enthaltend ein Glykosaminoglykan, das ausgewählt ist aus einer Gruppe bestehend aus Chondroitinsulfat, Keratansulfat, Dermatansulfat und Salzen davon, und mindestens einen nicht steroidalen entzündungshemmenden Wirkstoff, der ausgewählt ist aus einer Gruppe bestehend aus Indomethacin, Flurbiprofen, Naproxen, Diclofenac, Ketorolac, Mefenaminsäure, Ketoprofen, Meloxicam, Piroxicam, Nimesulid, Celecoxib, Rofecoxib, Etoricoxib, Parecoxib, Valdecoxib, Lumiracoxib, Licofelon und Salzen davon, optional mit unter pharmazeutischen Gesichtspunkten akzeptablen Hilfsstoffen, bei der Herstellung einer Zusammensetzung, die einen magenschonenden Effekt zur Verfügung stellt und die durch die Verabreichung von nicht steroidalen entzündungshemmenden Wirkstoffen hervorgerufene Gastrotoxizität minimiert.

## Revendications

1. Composition pharmaceutique comprenant un glycosaminoglycane choisi parmi un groupe constitué par le sulfate de chondroïtine, le sulfate de kératane, le sulfate de dermatane et les sels de ceux-ci, et au moins un médicament anti-inflammatoire non stéroïdien choisi parmi un groupe constitué par l'indométacine, le flurbiprofène, le naproxène, le diclofénac, le kétorolac, l'acide méfénamique, le kétoprofène, le méloxicame, le piroxicame, le nimésulide, le célécoxibe, le rofécoxibe, l'étoricoxibe, le parécoxibe, le valdécoxibe, le lumiracoxibe, le licofélone et les sels de ceux-ci, le cas échéant avec des excipients pharmaceutiquement acceptables, où ladite composition assure un effet gastro-protecteur et réduit la toxicité gastrique induite par l'administration dudit médicament anti-inflammatoire non stéroïdien.

2. Composition selon la revendication 1, dans laquelle le médicament anti-inflammatoire non stéroïdien est choisi parmi un groupe constitué par l'indométacine, le diclofénac, le nimésulide et les sels de ceux-ci.

3. Composition selon la revendication 1, dans laquelle le rapport du glycosaminoglycane ou des sels de celui-ci sur le médicament anti-inflammatoire non stéroïdien ou les sels de celui-ci varie de 100:1 à 1:100.

4. Composition selon l'une quelconque des revendications 1 à 3, dans laquelle les excipients pharmaceutiquement acceptables sont choisis parmi un groupe comprenant les diluants, les désintégrants, les agents de charge, les ingrédients de charge, les véhicules, les régulateurs de pH, les stabilisants, les anti-oxydants, les liants, les tampons, les lubrifiants, les anti-adhésifs, les agents d'enrobage, les émulsifiants, les agents de mise en suspension, les agents contrôlant la libération, les polymères, les colorants, les agents aromatisants, les plastifiants, les solvants, les agents de conservation, les régulateurs de glissement, les agents chélateurs utilisés soit séparément soit en association de tels agents.

5. Composition selon l'une quelconque des revendications 1 à 4, qui est formulée sous une forme galénique pour voie orale, une forme galénique pour voie pulmonaire, une forme galénique pour voie nasale, une forme galénique à usage topique, une forme galénique pour voie parentérale, une formulation à libération contrôlée, une formulation à dissolution rapide, une formulation lyophilisée, une formulation à libération retardée, une formulation à libération soutenue, une formulation à libération prolongée, une formulation à libération par impulsion ou une formulation mixte à libération immédiate et à libération contrôlée.

6. Composition selon la revendication 5, qui est formulée sous forme de comprimé, de pilule, de capsule, de gel, de poudre fine, de dispersion, de suspension, de solution ou d'émulsion.

7. Procédé pour préparer une composition pharmaceutique selon la revendication 1, qui comprend le mélange d'un glycosaminoglycane choisi parmi un groupe constitué par le sulfate de chondroitine, le sulfate de kératane, le sulfate de dermatane et les sels de ceux-ci, et d'au moins un médicament anti-inflammatoire non stéroïdien choisi parmi un groupe constitué par l'indométacine, le flurbiprofène, le naproxène, le diclofénac, le kétorolac, l'acide méfénamique, le kétoprofène, le méloxicame, le piroxicame, le nimésulide, le célécoxibe, le rofécoxibe, l'étoricoxibe, le parécoxibe, le valdécoxibe, le lumiracoxibe, le licofélone et les sels de ceux-ci, avec des excipients pharmaceutiquement acceptables et la formulation en une forme galénique appropriée.

8. Procédé selon la revendication 7, dans lequel le rapport du glycosaminoglycane ou des sels de celui-ci sur le médicament anti-inflammatoire non stéroïdien ou les sels de celui-ci varie de 100:1 à 1:100.

9. Utilisation d'une combinaison comprenant un glycosaminoglycane choisi parmi un groupe constitué par le sulfate de chondroïtine, le sulfate de kératane, le sulfate de dermatane et les sels de ceux-ci, et au moins un médicament anti-inflammatoire non stéroïdien choisi parmi un groupe constitué par l'indométacine, le flurbiprofène, le naproxène, le diclofénac, le kétorolac, l'acide méfénamique, le kétoprofène, le méloxicame, le piroxicame, le nimésulide, le célécoxibe, le rofécoxibe, l'étoricoxibe, le parécoxibe, le valdécoxibe, le lumiracoxibe, le licofélone et les sels de ceux-ci, le cas échéant avec des excipients pharmaceutiquement acceptables, dans la préparation d'une composition assurant un effet gastro-protecteur et réduisant la toxicité gastrique induite par l'administration du médicament anti-inflammatoire non stéroïdien.
